(19) Europäisches Patentamt
European Patent Office
Office européen des brevets

(11) Publication number: 0 262 941 A2

(12) # EUROPEAN PATENT APPLICATION

(21) Application number: 87308645.8

(22) Date of filing: 29.09.87

(51) Int. Cl.4: B 01 J 20/32
C 07 K 5/00, G 01 N 33/543

(30) Priority: 01.10.86 US 913901

(43) Date of publication of application:
06.04.88 Bulletin 88/14

(84) Designated Contracting States: ES GR

(71) Applicant: SMITHKLINE BECKMAN CORPORATION
One Franklin Plaza P O Box 7929
Philadelphia Pennsylvania 19103 (US)

(72) Inventor: Cantwell, Allan Michael
Deceased
Deceased (US)

Hill,Joe Marion
422 West Hortter Street
Philadelphia, Pennsylvania 19119 (US)

(74) Representative: Giddings, Peter John, Dr. et al
Smith Kline & French Laboratories Ltd. Corporate
Patents Mundells
Welwyn Garden City Hertfordshire AL7 1EY (GB)

(54) **Affinity purification process for glycopeptide antibiotics.**

(57) Glycopeptide antibiotics are selectively adsorbed by an adsorbent, A-B-C wherein A- is a carrier, -C is -D-alanine and -B- is a spacer having a hydrophilic substituent proximate to the -D-alanine.

EP 0 262 941 A2

## Description

Affinity Purification Process for Glycopeptide Antibiotics

### FIELD OF THE INVENTION

This invention relates to purification of glycopeptide antibiotics by use of a novel affinity ligand.

### BACKGROUND OF THE INVENTION

The glycopeptide, or vancomycin, class of antibiotics are crystalline, amphoteric, strongly levorotary compounds of relatively high molecular weight. Structurally, they comprise a polypeptide core aglycone structure having phenolic amino acids and one or more peripheral carbohydrate moieties. See, Williams et al., Topics in Antibiotic Chemistry, Volume 5, pages 119-158. Known members of this class include vancomycin (McCormick et al., U.S. Patent 3,067,099); ristocetin (Philip et al., U.S. Patent 2,990,329); A35512 (Michel et al., U.S. Patent 4,083,964; avoparcin (Kunstmann et al., U.S. Patent 3,338,786); teicoplanin (Bardone et al., J. Antibiot., Volume 31, page 170, 1978); actaplanin (Raun, U.S. Patent 3,816, 618); AAD-216 ("ardacin") (Bowie et al., U.S. Patent 4,548,974); A477 (Raun et al., U.S. Patent 3,928,571); OA7633 (Nishida et al., U.S. Patent 4,378,348); AM374 (Kunstmann et al., U.S. Patent 3,803,306); K288 (J. Antibiotics, Series A, Volume 14, page 141 (1961), also known as actinoidin).

Glycopeptide antibiotics generally are produced in complexes of one or more major components and one or more minor components, which share the same or an analogous core aglycone. Glycopeptides can be hydrolyzed to remove one or more carbohydrate moieties usually without adversely affecting antibiotic activity.

The glycopeptide antibiotics exhibit antibacterial activity, some having therapeutic uses against gram-positive organisms. In addition, many have been demonstrated to increase animal feed utilization efficiency, to be useful to promote animal growth, to improve milk production in ruminants and to treat and to prevent ketosis in ruminants. For example, Reynolds et al., GB2137087A, disclose use of avoparcin to improve milk production; Raun et al., U.S. Patent 3,928,571 disclose use of certain glycopeptide antibiotics to promote growth and to prevent and to treat ketosis; Hamill et al., U.S. Patent 3,952,095, disclose use of actaplanin to promote growth; and Ingle et al., U.S. Patent 4,206,203 disclose use of avoparcin to prevent and to treat ketosis.

References which describe purification of one or more glycopeptide antibiotics include Sitrin et al., EP-A-132,117, who disclose use of -D-alanyl-D-alanine or -X-D-alanyl-D- alanine, wherein X can be lysine, as an affinity ligand. Corti et al., Applied Biochem. Biotech. 11:101(1985), and Cassani et al., EP-A-122,969, who disclose use of certain affinity adsorbents generically represented by the formula:

A-B-R-CO-D-ala-Z

wherein

A is a carrier;

B is a bond, spacer arm or polyaminoacidic residue;

R is a straight or branched $C_{1-12}$ alkylene amino- or oxy- group of the formula -HN($C_{1-12}$ alkylene) or -0($C_{1-12}$ alkylene) or R-CO is L-lysinyl, a bond or a group -OC($CH_2$)$_n$ CO- wherein n is 1-6; and

Z is D-ala, glycinyl or D-leucinyl; such that the -D-ala-moiety is bonded through its amino function to a carboxy function contributed by -R-CO-, -B- or A-.

### SUMMARY OF THE INVENTION

It has now been discovered that glycopeptide antibiotics are efficiently adsorbed to an affinity adsorbent comprising a solid carrier matrix and a ligand which is -D-alanine coupled to the matrix through a spacer having at least one hydrophilic substituent proximate to the -D-alanine.

More particularly, the invention is an affinity adsorbent for selective adsorption of glycopeptide antibiotics which has the formula A-B-C wherein

A is a solid carrier;

B is an organic spacer having at least one hydrophilic substituent proximate to the -D-alanine; and

C is -D-alanine attached to B through its amino function.

These affinity adsorbents and preferred embodiments thereof, as well as their production and use, all of which are considered as further aspects of the same invention, are fully described herein below.

### DETAILED DESCRIPTION OF THE INVENTION

An affinity purification process makes use of a ligand which can selectively bind to a substance to be separated from a solution thereof. Such ligand must be capable of chemical and physical ("lock and key") interaction with the substance. The affinity must be sufficiently strong so as to adsorb the substance, but must also be reversible to permit subsequent desorption and elution without denaturation of the substance. The affinity must also be sufficiently selective so as not to adsorb significant amounts of impurities.

The present invention employs use of -D-ala and a spacer having a hydrophilic substituent proximate to the -D-ala moiety such that the ligand is specifically interactive with glycopeptide antibiotics. Such ligand, therefore, sufficiently mimics the physical and chemical properties of -lys-D-ala-D-ala and -D-ala-D-ala affinity ligands for glycopeptides so as to provide the ligand with sufficient affinity for glycopeptide antibiotics and

thereby to permit use of the ligand in an affinity process for purifying glycopeptide antibiotics from an impure solution thereof.

Initial results of experiments using alternatively, -D-ala, -L-ala, -gly and (D,L-) lactic acid, all of which possess similar chemical structures differing only in the functional group attached to the Cl carbon of the carboxylic acid or in the configuration around the asymmetric Cl carbon atom in the case of L-ala, showed that D-ala differed from the ligand analogs in the mechanism of binding. All prepared ligand adsorbents possessed the ability to nonspecifically adsorb ardacin from crude preparations. For example, as shown in Table 1 below, the D-ala ligand adsorbs ardacin from crude preparations to a concentration of 2.8 mmoles ardacin/L of adsorbent and D-ala-D-ala adsorbs 3.6 mmoles ardacin/L of adsorbent, whereas the analogs of L-ala, D,L-lactate, and glycine adsorbed 1.3, 0.8, and 0.9 mmoles ardacin/L adsorbent respectively. When the loaded adsorbents were washed with 0.1 M phosphate, pH 7.0 buffer, the ardacin was largely washed off the ligand analogs L-ala, D,L-lactate, and gly along with the non-specifically adsorbed impurities present in the crude feed which were also non-affinity adsorbed during the loading. In comparison, the D-ala-D-ala and D-ala ligands retained the bulk of the adsorbed ardacin during the phosphate washing step and only non-affinity adsorbed impurities were removed at this stage. Finally, a desorbing buffer (50% acetonitrile in 0.1M phosphate, pH 7.0) was used to desorb the ardacin from the adsorbent by breaking up the hydrogen bonding interactions between the ligand and ardacin, responsible for the affinity adsorption. In the case of the D-ala ligand, 81 percent of the ardacin loaded was recovered in the desorbed fraction at a purity of 92 percent. The non-affinity adsorbents were already depleted of ardacin by the phosphate wash. Therefore, only 22 to 15 percent of the total ardacin loaded was recovered during desorption and the purity of the fraction was only 37 to 51 percent. Thus, due to the absence of specific affinity adsorption, ardacin is not purified by the non-affinity ligands.

The results reported in Table 1 were produced in an experiment in which silica ($100 \times 10^{-6}$m diameter), 280 angstrom ($280 \times 10^{-10}$m) pore size was coupled, via a $(CH_2)_3$ spacer and carbonyl-diimidazole activation, to various ligands (150 mM) to prepare an affinity adsorbent: $A(CH_2)_3$ NHCO-D wherein A is the silica carrier and D is the ligand. Ardacin (0.2 mg/ml) previously purified from a fermentation broth by adsorption on HP-20 substantially as described by McCormick et al., U.S. Patent 4,440,753, to about 50% purity by weight, was applied to the column in 0.1 M phosphate, pH 7.0, until breakthrough. The column was washed with the same buffer until baseline absorbance at 280 nm was achieved. Elution was by washing with 50:50, 0.1 M phosphate:acetonitrile. Maximum loading is defined as the concentration (mM) of ardacin on the adsorbent at the point of breakthrough. Percent recovery is the amount of ardacin desorbed from the column following the wash divided by the maximum loading. Percent purity is of the desorbed fraction and was determined by HPLC.

Table 1

| LIGAND | MAXIMUM LOADING (mM) | % RECOVERY | % PURITY |
|---|---|---|---|
| D-Ala-D-Ala | 3.6 | 76 | 95 |
| D-Ala | 2.8 | 81 | 92 |
| L-Ala | 1.3 | 20 | 40 |
| D-Lactate | 0.8 | 22 | 37 |
| Glycine | 0.9 | 15 | 51 |

The results shown in Table 1, as discussed above, reveal that only the D-ala and the D-ala-D-ala ligands retained most of the antibiotic during the washing step and yielded a highly purified antibiotic fraction upon desorption. The D-ala analogs showed low retention of the antibiotic during the wash step and the desorption of the antibiotic yielding in some instances antibiotic of lower purity than the starting material. Also, the appearance of the desorbed product, after lyophilization, was dramatically different. Rather than the light, fluffy, off-white powder produced by the D-alanyl-D-alanine and the D-alanine affinity adsorbents, the alanine analogs yielded an amorphous, dark, granular solid. Thus, the ligand of the invention, can be used to recover greater than 70% of a glycopeptide antibiotic previously purified on HP-20 to about 50% purity by weight, the recovered antibiotic being at least about 85%, preferably at least about 90%, pure. All (100%) of the antibiotic can ultimately be recovered by recycling the phosphate washes.

Data of an experiment comparing adsorption and desorption on D-ala affinity silica with $C_3$ or $C_6$ alkyl spacer arms and with a $C_6$ hydroxy ether, $(CH_2)_3O$ $CH_2CH(OH)CH_2$-, spacer arm are reported in Table 2, below. The adsorption and desorption were carried out substantially as described above.

Table 2

| SPACER ARM | MAXIMUM LOADING (mM) | % RECOVERY | % PURITY |
|---|---|---|---|
| C3 | 2.8 | 81 | 92 |
| C8 | 3.2 | 68 | 85 |
| C6 HYDROXYETHER | 3.1 | 97 | 98 |

These results show that introduction of a hydrophilic group in the spacer, in this case -OH, enhances the performance of the affinity adsorbent. Other hydrophilic substituents, which are nucleophiles capable of hydrogen bonding with the heptapeptide core of a glycopeptide antibiotic can also be used. These include, for example, oxygen, sulfhydryl, $C_{1-4}$ thioalkyl and amino. While not intending to be bound to a theory as to mechanism of action, it appears that the hydrophilic substituent replaces the affinity contributed by -D-alanyl- in a -D-alanyl-D-alanine ligand.

The carrier to which the affinity ligand is coupled to prepare the affinity adsorbent of the invention can be any material which is insoluble in the buffers employed and which is capable of chemically coupling the ligand. Many such carriers are known. These include glass, silica, alumina and zirconia as well as organic carriers such as agarose, cellulose, dextran, polyamide, polyacrylamide and vinyl copolymers of bifunctional acrylates with various hydroxylated monomers. Commercially available carriers include, for example, Affi-gel®, Sephadex®, LiChrosorb®, Trisacryl®, Cellufine™, Biogel™, Acrobead™, Excellulose™ and Matrex™.

A suitable carrier, which exhibits minimal interaction with the glycopeptide and with other solute materials which may be present in impure glycopeptide preparations, can be selected by standard techniques. Such matrix of carrier materials preferably has sufficient porosity to permit ready diffusion of solutes to the adsorbent surface.

Silica is a preferred carrier. Commercially available silica suitable for preparative affinity applications is available in pore sizes from 60 A to 300 A (60 to 300 $\times$ $10^{-10}$m). Affinity adsorbents based on silica of 60, 150, and 280 angstroms (60, 150 and 280 $\times$ $10^{-10}$m) pore size were prepared. Column adsorption results using ardacin, shown in Table 3, show a pronounced effect of pore size on adsorbent capacity.

Additional preliminary work using ardacin with an experimental 500 A (500 $\times$ $10^{-10}$m) pore silica indicates that this trend continues. However, at pore sizes above 500 A (500 $\times$ $10^{-10}$m), the surface area of the silica diminishes rapidly and thus the ligand densities found to give the highest loadings can no longer be achieved. Thus, pore sizes of 200 A to 500 A (200 to 500 $\times$ $10^{-10}$m) would appear to be the best range.

Table 3

| Silica Pore Size ($x10^{-10}$m) | Ardacin-Initial Loading (moles $x10^{-6}$) | Ardacin Adsorbed (moles $x10^{-6}$) | Ardacin Un-Adsorbed (moles $x10^{-6}$) |
|---|---|---|---|
| 60 | 10 | 0 | 10 |
| 60 | 100 | 0.2 | 100 |
| 150 | 10 | 3.5 | 6.5 |
| 150 | 100 | 1.0 | 99 |
| 280 | 10 | 10 | 0 |
| 280 | 100 | 3.0 | 97 |

The results reported in Table 3 were obtained as follows. D-Alanine ligand concentration of 150 mM was coupled to silica via a $C_3$ alkyl spacer arm and carbonyldiimidazole activation. 5 mL of the affinity adsorbent was treated with 100 mL of the initial ardacin solution. Desorption was by 50:50 0.1 M phosphate, pH 7.0: methanol. Concentrations of ardacin adsorbed were measured by analytical HPLC of the desorbed peak.

Concentration of ardacin not adsorbed was measured by analytical HPLC of the solution eluting from the column during sample application.

The spacer, -B-, contributes to the affinity of the adsorbent of the invention for glycopeptide antibiotics. It is a $C_2$ - $C_{12}$ alkylene substituted with a hydrophilic moiety proximate to the alanine, that is, within 3 carbon atoms of the alanine. Preferred hydrophilic substituents are =0, -OH, -SH, -$SC_{1-4}$ alkyl and -$NH_2$ at positions 1, 2 or 3 relative to the amino terminus of the alanine. The alkylene spacer may contain one or more in-chain hetero atoms between alkylene carbon atoms. For example, the alkylene chain can be interspersed with one or more of -0- and -NH-. Selection of -B- will depend, in part, upon choice of the solid carrier. Thus, for example, when A- is silica, B is a mono-, di- or trifunctional organosilane. Examples of suitable groups -B- are, among others,

$A - CH_2 - CH(OH) - CH_2-;$

$A - CH(OH) - CH_2 - CH_2-;$

$A -(CH_2)_3CH(SH)-;$

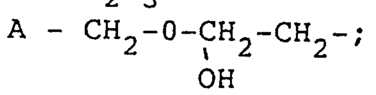

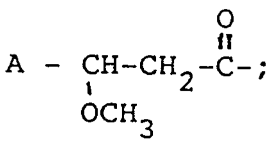

$A - CH_2CH(CH_3)CH(OH)-.$

Examples of preferred groups, -B-, when A is silica include:

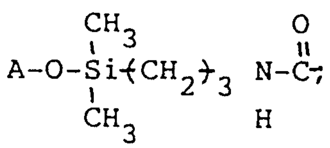

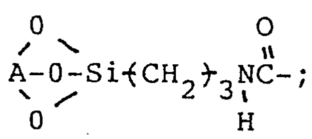

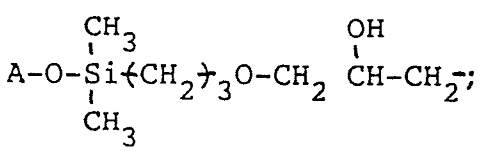 and

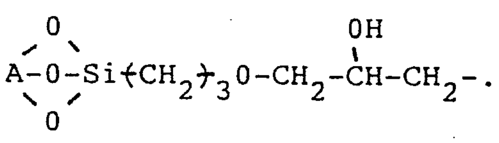

The affinity adsorbent of the invention is useful in selectively adsorbing glycopeptide antibiotics in batch or chromatographic fashion from an impure solution thereof. The impure solution of glycopeptides can be a crude fermentation broth, but is preferably a partially purified fermentation broth. Such prior partial purification can be achieved by standard techniques. These include adsorption such as on a non-functional resin as disclosed by McCormick et al., U.S. Patent 4,440,753, or other HPLC procedures. Preferably the impure solution is about 50% pure by weight of solids.

The affinity ligand is coupled to the carrier by standard techniques. The following Scheme A and Scheme B are illustrative of carbonyldimidazole (CDI) coupling of D-alanine to a spacer previously bound to silica.

Scheme C illustrates epoxide coupling of D-alanine directly to the spacer group.

(Me is $-CH_3$, R is $-\overset{OCH_3}{\underset{OCH_3}{Si}}-(CH_2)_2$ and Et is $-CH_2-CH_2-$.

Scheme A (I)

CDI COUPLING - $C_3$ ALKYL SPACER - MONOFUNCTIONAL SILANE

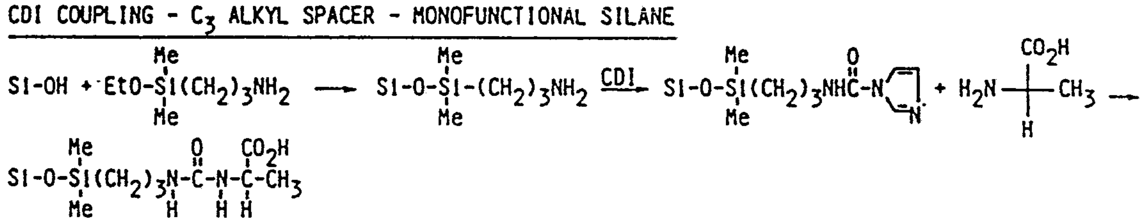

Scheme A (II)

EPOXIDE COUPLING - $C_6$ HYDROXY ETHER SPACER - MONOFUNCTIONAL SILANE

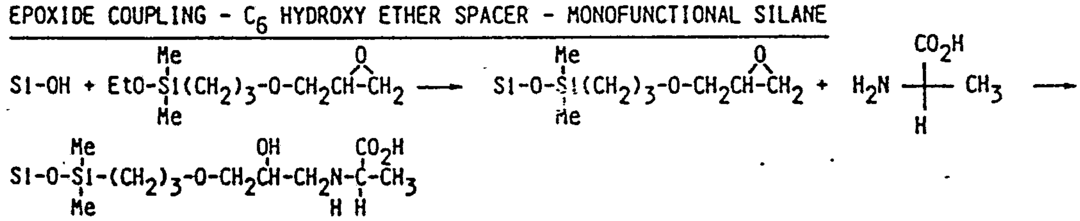

Scheme B

CDI COUPLING - $C_3$ ALKYL SPACER - TRIFUNCTIONAL SILANE

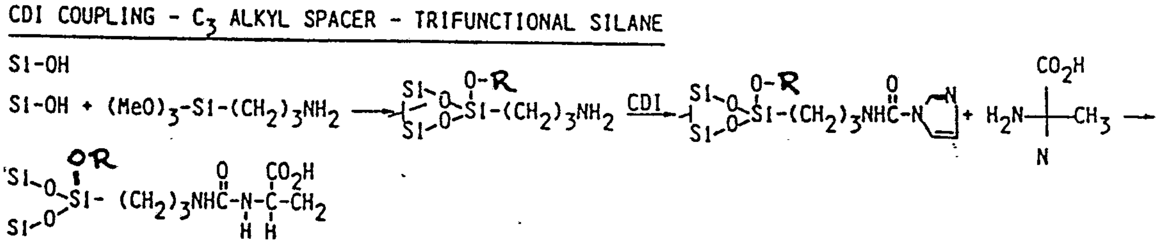

Using such processes, ligand densities up to 150 mM can be achieved on silica. In Table 4, below, results of three ligand densities are compared. At the highest ligand density of 150 mM the recovery of ardacin was lower (81% vs 96%) than the 17 mM ligand density adsorbent. Product purity was also reduced somewhat. The optimal ligand density for the purification of ardacin on D-alanine silica thus appears to be in the 5 to 20 mM range. It should be noted, however, that higher ligand densities may be effective with other glycopeptide antibiotics. Ardacin may not be able to fully utilize a high ligand density surface. Glycopeptides not having a lipid moiety may be adsorbed to higher loadings on high ligand density silica.

Table 4

| SILICA LIGAND DENSITY (mM) | MAXIUMUM LOADING (mM) | % RECOVERY | % PURITY |
|---|---|---|---|
| 3 | 1.7 | 93 | 98 |
| 17 | 2.3 | 96 | 98 |
| 150 | 2.8 | 81 | 92 |

The results reported in Table 4 were obtained using 100 um, 280 A (280 × $10^{-10}$m) pore size silica with a C3 spacer which was; CDI-coupled to D-ala; 0.2 mg/ml of ardacin was applied to the column in 0.1 M phosphate (pH 7.0) until breakthrough; Elution was by 50:50 0.1M phosphate:acetonitrile. Purity determination was by analytical HPLC.

Buffer systems are selected to optimize selective adsorption and desorption. Buffer systems employed for the adsorption and desorption onto known affinity supports, e.g., -D-ala-D-ala-Affi-gel® can be used for the affinity process of this invention. Thus, teicoplanin can be desorbed using 0.15 M NaCl, 0.05 or sodium phosphate buffer (pH 11), as reported by Corti et al., Appl. Biochem. Biotech, 11:101(1985); Sitrin et al., EP-A-132,117 disclose use of 10 - 30% acetonitrile, bicarbonate buffer (PH 9-9.5) to desorb various glycopeptides. When silica is the carrier, pH > 8 should be avoided due to degradation of the silica.

Prior to loading, the solution of glycopeptide antibiotic is preferably admixed with an adsorption buffer. It was found that 0.1 M phosphate, pH 7.0 increases the capacity of silica for ardacin. This improvement in capacity was enhanced further by the addition of 1 M NaCl to the adsorbing buffer and wash buffer. Desorption is accomplished by switching to a buffer of 50% 0.1 M phosphate (pH 7.0):50% methanol. A buffer of 80% 0.1 M D-ala, (pH 3.5), 20% acetonitrile can also be used to desorb ardacin from a silica affinity support.

The following examples are illustrative, and not limiting, of methods of preparing the affinity adsorbent of the invention and use of such adsorbent to selectively adsorb glycopeptide antibiotics.

EXAMPLE 1: Preparation of D-Alanine Affinity Chromatography adsorbent

A 100 g portion of silica is stirred into 500 mL of 4 N hydrochloric acid. The slurry is heated to 104°C for two hours and then cooled by slowly adding 2L of distilled water. The slurry is settled and the supernatant is decanted. Distilled water (2L) is added to the settled silica and resuspended, settled, and decanted repeatedly until the pH of the decanted water is greater than 5.0. This typically requires five cycles. The silica is drained and dried in vacuo at 150°C to constant weight.

The dried silica is added with stirring to 1750 mL of anhydrous toluene. Five grams of 3-glycidoxypropyldimethyl ethoxy silane is added dropwise to the slurry. The temperature is increased to reflux (105°C) and the reaction solution is maintained at this temperature for five hours. The reaction solution is slowly sparged with dry nitrogen during this period. The reaction solution is then cooled and the derivatized silica is filtered. The activated silica is washed with toluene by the slurry and filtration technique until traces of unreacted epoxide are not detectable by the thiosulfate test in the wash solvent. The activated silica is dried in vacuo at 20°C to constant weight.

The amount of activated silica to be coupled with the D-alanine is weighed out. The activated silica is added to a buffer solvent of acetonitrile containing 5 percent by weight distilled water, and 1 percent by weight 1 M potassium phosphate, pH 7.5. The solution should contain 1 g of activated silica per 50 mL of buffer solvent. The slurry is degassed. D-alanine dissolved in the buffer solvent is added at a rate of 70 mg D-alanine per g of activated silica. The slurry is agitated on a shaker for 24 hours at room temperature. A chromatographic column is packed with the D-alanine-silica and rinsed with ten column volumes of distilled, degassed water prior to use, or until 215 nm absorbance of the wash water is not increased.

EXAMPLE 2: Affinity Chromatography of Ardacin On D-Alanine Silica

An affinity adsorbent prepared according to the procedure of Example 1 was loaded into a 3 mL (0.7 × 8 cm) chromatographic column and equilibrated with 0.2 M sodium phosphate, pH 7.0, 1.0 M sodium chloride. A sample of partially purified (53 percent purity w/w) ardacin dissolved in the equilibrating buffer at a concentration of 0.2 mg of ardacin per mL of buffer was applied to the column until a total of 5 mg of ardacin had been introduced. The column was then washed with the equilibrating buffer until the absorbance of the eluant at 280 nm had returned to baseline (approximately 30 mL). A desorbing buffer of 50:50, 0.2 M sodium phosphate, pH 7.0:methanol was applied to the column. The eluting fraction exhibiting 280 nm absorbance was collected (3 mL). The fraction was assayed by analytical HPLC against a reference standard as 98 percent ardacin. The fraction was desalted by Trisacryl® GF05 gel filtration chromatography and lyophilized to yield 4.2 mg of ardacin.

EXAMPLE 3: Preparation of D-Alanine Affinity Chromatography Adsorbent

A 100 g portion of 280 × $10^{-10}$m pore size, 60 um particle size silica is stirred into 500 mL of 4N hydrochloric acid. The slurry is heated to 104°C for two hours and then cooled by slowly adding 2L of deionized water. The slurry is settled and the supernatant decanted repeatedly until the pH of the decanted water is greater than 5.0 (approximately 25 silica volumes). The silica is drained and dried in vacuo at 150°C to constant weight. This typically requires 36 hours.

The dried, acid-treated silica is added to 500 mL of dry toluene in a suitable vessel fitted with a paddle stirrer, addition funnel, and condensor with drying tube. While stirring, 13.3 g of aminoproplytriethoxysilane diluted with 100 mL of toluene is added to the silics slurry over a two hour period. Following addition, the temperature is increased to reflux (105°C), and the slurry is refluxed eight hours.

The slurry is cooled and settled. The supernatant is decanted and the solids are washed two times with 500 mL of toluene by the mixing-settling method.

To the toluene-saturated solids are added 400 mL of dry toluene and 10.3 g of dry pyridine diluted with 100 mL of toluene. While stirring, the slurry is rinsed with demineralized water until a test for amine in the eluant is negative. The affinity adsorbent is then ready for use.

Thirteen g of trimethylchlorosilane is diluted with 100 ml of dry toluene over 30 minutes. The mixture is heated to reflux (105°C) and refluxed one hour. The slurry is cooled and settled. The supernatant is decanted and the solids are washed five times with 500 mL of toluene by the mixing-settling method.

To the toluene saturated solids are added 100 mL of dioxane. The mixture is slurried, and then settled and decanted three times. To the dioxane saturated solids are added 200 mL of dioxane. Then, 9.7 gm of carbonyldiimidazole are added to the stirred slurry and the slurry is mixed for 45 minutes. The supernatant is decanted and the solids are washed with 100 mL of dioxane. The wash step is repeated five times.

D-Alanine (6.1 gm) is dissolved in 200 mL of 0.1 M ammonium bicarbonate buffer, pH 8.0. The alanine solution is added to the dioxane saturated silica and stirred for eight hours. The supernatant is decanted and the solids are washed by using the mixing-settling method with 800 mL of 0.1 M ammonium bicarbonate, pH 8.0.

500 mL of 0.1 M ethanol amine buffer, pH 8.0 are added to the solids with stirring and mixed for 2 hours. The slurry is then poured into a chromatographic column.

EXAMPLE 4: Affinity Chromatography of Vancomycin On D-Alanine Silica Affinity Adsorbent

An affinity adsorbent prepared by the procedure of Example 3 was loaded into a 25 mm diameter chromatography column, total bed volume 125 mL. Vancomycin hydrochloride (Sigma Chemical Co., St. Louis, Missouri, U.S.A.) assayed by analytical HPLC at 91 percent by area was dissolved in 0.1 M sodium phosphate, pH 3.6, 1.0 M sodium chloride to prepare a solution of 2 mg/mL. The vancomycin solution was applied to the affinity adsorbent column until breakthrough of vancomycin in the column eluant was detected. The affinity column was then washed with 0.1 M sodium phosphate, pH 3.6 until no vancomycin was detected in the column eluant. At this point an estimated 1.09 g of vancomycin•HCl (100% purity basis) had been loaded on the affinity column. A desorbing buffer of 50:50, 0.1 M sodium phosphate, pH 3.6-methanol was applied to the column. The fraction exhibiting 280 nm absorbance was collected (88 mL) and assayed by analytical HPLC. The solution had a concentration of 11.3 mg vancomycin/mL or a total recovery of 997 mg of vancomycin and a purity of 96 percent by HPLC area.

The above descriptions and examples fully disclose this invention and preferred embodiments thereof. The invention is not limited to constructions specifically exemplified herein but, rather, includes all embodiments coming within the scope of the following claims.

## Claims

1. An affinity adsorbent for selective adsorption of a glycopeptide antibiotic from an impure solution thereof having the formula A-B-C wherein

A is a solid carrier;

B is an organic spacer having at least one hydrophilic substituent proximate to the D-alanine and

C is -D-alanine.

2. The affinity adsorbent of Claim 1 wherein B is $C_{2-12}$ alkylene substituted with a hydrophilic moiety selected from the group consisting of =0, -OH, -SH, $SC_{1-4}$ alkyl or $-NH_2$ at positions 1, 2 or 3, relative to the -D-alanine and wherein the alkylene chain is optionally interspersed with -0- or -NH-.

3. The adsorbent of 1 selected from the group consisting of

$A - CH_2 - CH(OH) - CH_2-;$

$A - CH(OH) - CH_2 - CH_2-;$

$A -(CH_2)_3CH(SH)-;$

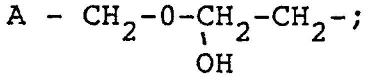

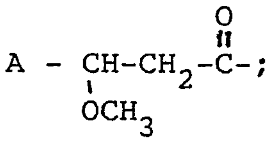

$A - CH_2CH(CH_3)CH(OH)-.$

4. The adsorbent of Claim 1 wherein A is silica having average pore size of 60 to 500 A (60 to 500 × $10^{-10}$m).

5. The adsorbent of Claim 4 selected from the group consisting of

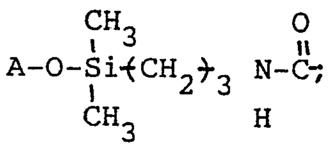

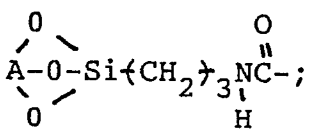

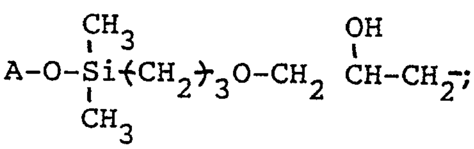 and

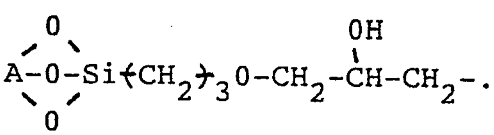

6. A method for selectively separating a glycopeptide antibiotic from an impure solution thereof which comprises contacting the impure solution with an affinity adsorbent having the formula A-B-C wherein

A is a solid carrier;

B is an organic species having at least one hydrophilic substituent proximate to the D-alanine and

C is -D-alanine.

7. The method of Claim 6 wherein B is $C_{2-12}$alkylene substituted with a hydrophilic moiety selected from the group consisting of =0, -OH, -SH, $SC_{1-4}$ alkyl or $-NH_2$ at positions 1, 2 or 3, relative to the -D-alanine and wherein the alkylene chain is optionally interspersed with -0- or -NH-.

8. The method of Claim 7 wherein the adsorbent is selected from,

$A - CH_2 - CH(OH) - CH_2-$;

$A - CH(OH) - CH_2 - CH_2-$;

$A -(CH_2)_3-CH(SH)-$;

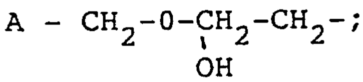

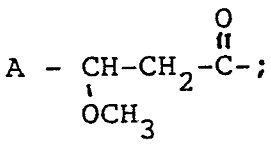

$A - CH_2CH(CH_3)CH(OH)-$.

9. The method of Claim 6 wherein A is silica having average pore size of 60 to 500 A (60 to 500 × $10^{-10}$m).

10. The method of Claim 9 wherein the adsorbent is selected from,

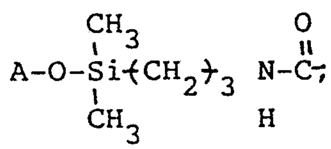

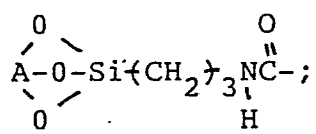

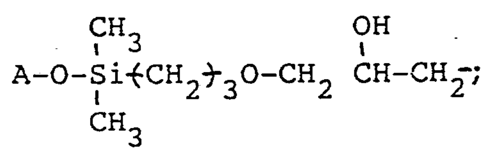 and

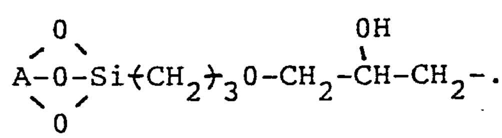

11. The affinity adsorbent of claim 1 having a glycopeptide antibiotic bound thereto.